# EUROPEAN PATENT APPLICATION

(11) **EP 4 115 816 A1**
(43) Date of publication of application: **11.01.2023**
(21) Application number: 22182875.9
(22) Date of filing: 04.07.2022
(51) Int. Cl.: A61B 10/00, B01L 3/00, A61B 10/02

(54) **DEVICE AND METHOD FOR TAKING AND HANDLING A SALIVA SAMPLE**

(30) Priority: 06.07.2021 IT 202100017780
(71) Applicant: Universita' degli Studi di Padova, 35137 Padova (IT)
(72) Inventor: Basso, Daniela, 30020 Pianiga (IT); Recchia, Francesco, 31100 Treviso (IT); Soramel, Francesca, 35133 Padova (IT); Lenzi, Silvia Monica, 35127 Padova (IT); Plebani, Mario, 35123 Padova (IT); Aita, Ada, 35125 Padova (IT); Padoan, Andrea, 35124 Padova (IT)
(74) Representative: Busana, Omar

(57) **Abstract**

A device (12) for taking and handling a saliva sample comprises: a test tube (14) suitable for containing a sample of saliva; a collection element (16) comprising a stem (20) provided at a first end (202) with an absorbent element (22) suitable for absorbing a specified amount of saliva, and suitable for being squeezed to release at least part of the amount of saliva absorbed. The device further comprises a collector (24) provided at a first end (242) with a mouth (244) of a shaped seat (26) suitable to allow the insertion of said absorbent element (22), said shaped seat (26) comprising a narrowing (28) suitable for allowing the squeezing of said absorbent element (22); said collector (24) at a second end (246) comprising at least one collection hole (30, 31). The collector (24) is suitable for being at least partially inserted inside the test tube (14) so that said at least one collection hole (30, 31) is suitable for realizing a fluidic communication between the shaped seat (26) and the inside of the test tube (14).

## Description

### FIELD OF APPLICATION

The present invention relates to a device and a method for taking and handling a saliva sample. In particular, the present invention relates to a device and a method for self-collecting and handling a saliva sample.

### PRIOR ART

As is known, saliva is a biological sample suitable for searching for multiple molecular and biochemical analytes. For example, it is recognized as the most suitable sample for determining the hormones that regulate the circadian rhythm, i.e. cortisol and melatonin.

Recently, the search for viral sequences on saliva samples has been validated as a high-sensitivity and specificity instrument, completely comparable to the search with nasopharyngeal swab.

For this reason, in the search for the SARS-CoV-2 virus, saliva diagnostics has emerged as an alternative method, especially in the screening of asymptomatic subjects.

In addition, saliva samples are also used to diagnose HIV-induced diseases and many other viruses, and to detect illicit drug use, and numerous studies in the literature also support the use of saliva samples for the search for molecular and biochemical biomarkers in oncology, rare diseases and chronic diseases.

The main advantage in using the saliva matrix for the diagnosis and monitoring of diseases compared to blood tests is that the sample collection may be carried out in a non-invasive and painless manner without the need for specific skills. In some cases, this task may also be carried out directly and independently as a self-diagnosis.

Therefore, the analysis of a saliva sample lends itself by its nature to being a suitable technique also for large-scale screening programs.

In the prior art there are various methods of collecting human saliva samples based on direct collection by spitting, aspiration or swab absorption. In particular, saliva collection devices are known which allow the collection of saliva and which may be used for taking stimulated or non-stimulated saliva.

In a first example, the device consists of a rectangular cellulose swab attached to a plastic stem. The cellulose material is rubbed onto the cheek surfaces adjacent to the gum for a specified time and then left in the oral cavity between the teeth and the gum to absorb the saliva sample. Subsequently, the cellulose material, and therefore the saliva sample, is placed in a test tube containing a solution and is then transported to the laboratory for all the following analysis steps.

In a second example, the device comprises a paper-based cellulose material attached to a stem for collecting saliva from the mouth. The absorbent swab is placed in the mouth and saliva is collected until a sample volume indicator, which is integrated into the device, changes color from white to blue (usually about 2 minutes) indicating that there is sufficient saliva (1.0 ml). The absorbent swab has a series of perforations near the upper part of the cellulose swab, which allows the swab to detach. The transport test tube contains a stabilizing solution to ensure sample preservation for laboratory testing.

A third example relates to a device, available either as cotton in roll form or a polyester sponge with a sample transport test tube included. In order to collect a sample, the device is placed in the mouth and chewed for about two minutes. It is then placed in the transport test tube for transport to the laboratory.

A fourth example relates to an apparatus consisting of two collection test tubes connected to a single mouthpiece in which the user expectorates. The mouthpiece is rotated during collection to direct saliva from one of the two tubes to the other. The test tubes are then transported to the analysis laboratory.

A fifth example relates to a funnel-shaped plastic device in which to expectorate/spit saliva. The funnel is connected directly to a manufacturer-supplied transport test tube which is used for transport to the laboratory.

In a sixth example, an apparatus uses an absorbent material in the form of a swab to collect 1 ml of whole saliva. The kit consists of an absorbent foam pad, a centrifuge test tube and a stopper. In order to collect saliva, the swab is placed in the mouth for a specified time. Subsequently, in the laboratory the sample is removed from the swab by centrifugation.

In a seventh example, a kit associated with a method is proposed, wherein: the mouth is rinsed with a reagent, a 4 ml sample of a tartrazine solution is kept in the mouth for 2 minutes and then the entire contents are spit out in a test tube containing preservative agents.

In an eighth example, an absorbent swab made of non-cellulose material is used to collect saliva from under the tongue until an indicator on the apparatus signals that an adequate sample volume has been collected. Typical collection time is around 1-2 minutes. The saliva is then separated from the absorbent collection swab by squeezing, compressing the walls of a test tube with a soft plastic section, then passing the saliva sample into a test tube suitable for transport.

For the specific case of SARS-COV-2, to which however the invention is not limited, RNA is stable in saliva for a wide range of temperatures and for a prolonged period of time, eliminating the need for preservatives.

However, the saliva collection methods and devices of the prior art, although appreciated, are not free from drawbacks.

For example, in some cases, the devices of the prior art are made with materials which may bind specific molecules, leading to a limited recovery and therefore to a clinical risk due to the imprecision and inaccuracy of the evaluation of the results.

Furthermore, improperly collected saliva poses handling difficulties in the laboratory. This problem is particularly relevant in the case in which the self-collection is carried out by children or people with limited manual skills. When not carried out with suitable means, self-collection may also pose a safety risk if the materials having a biological risk are not properly treated. Therefore, it is essential that the self-collection of saliva is safe and may produce reproducible samples suitably translatable into quantitative measurements. Equally important is the acceptability of self-collection for the public and therefore methods that may be deemed as inconvenient or difficult should be avoided as they would not have the prospect of achieving the appropriate diffusion amongst the population.

In fact, a properly carried out collection may simplify the subsequent analysis steps in the laboratory, for example avoiding centrifugation to extract the sample from the swab, or be suitable for an analysis carried out with rapid self-diagnostic tests.

### DISCLOSURE OF THE INVENTION

There is therefore a need to resolve the drawbacks and limitations mentioned with reference to the prior art.

Therefore, the need is felt to provide a device and a method for taking and subsequently handling a saliva sample which allows easy taking of the sample and which may possibly be done independently by the person him/herself.

Furthermore, the need is felt for a device and a relative method which may be used autonomously also by a child or by a person with limited mobility.

Furthermore, the need is felt for a device and a relative method which allows the saliva to be collected in a container which may be directly used by any analysis laboratory.

Furthermore, the need is felt for a device and a relative method which allows the collection of the saliva sample to be carried out, preventing any external contamination of the container.

Furthermore, the need is felt for a device and a relative method for collecting a saliva sample which is easy to perform and easily accepted by the person.

These needs are at least partially met by a device for taking and handling a saliva sample according to claim 1, and by a method for taking and handling a saliva sample according to claim 13.

### DESCRIPTION OF THE DRAWINGS

Further features and advantages of this invention will become more apparent from the following detailed description of preferred, non-limiting embodiments thereof, in which:
- Fig. 1 shows schematically a perspective view of a device according to a possible embodiment of the present invention;
- Fig. 2 shows schematically a perspective view of a test tube of a device according to an embodiment of the present invention;
- Fig. 3 shows schematically a perspective view partly in cross section of an element of a device according to an embodiment of the present invention;
- Fig. 4 shows schematically a perspective view partly in cross section of an element of a device according to an embodiment of the present invention;
- Fig. 5 shows schematically a perspective view of an element of a device according to an embodiment of this invention;
- Fig. 6 shows schematically a device according to an embodiment of the present invention, in a possible configuration of use;
- Fig. 7 shows schematically a device according to an embodiment of the present invention, in a possible configuration of use; and
- Fig. 8 shows schematically a device according to an embodiment of the present invention, in a possible configuration of use.

Elements or parts of elements common to the embodiments described hereinafter will be indicated with the same reference signs.

### DETAILED DESCRIPTION

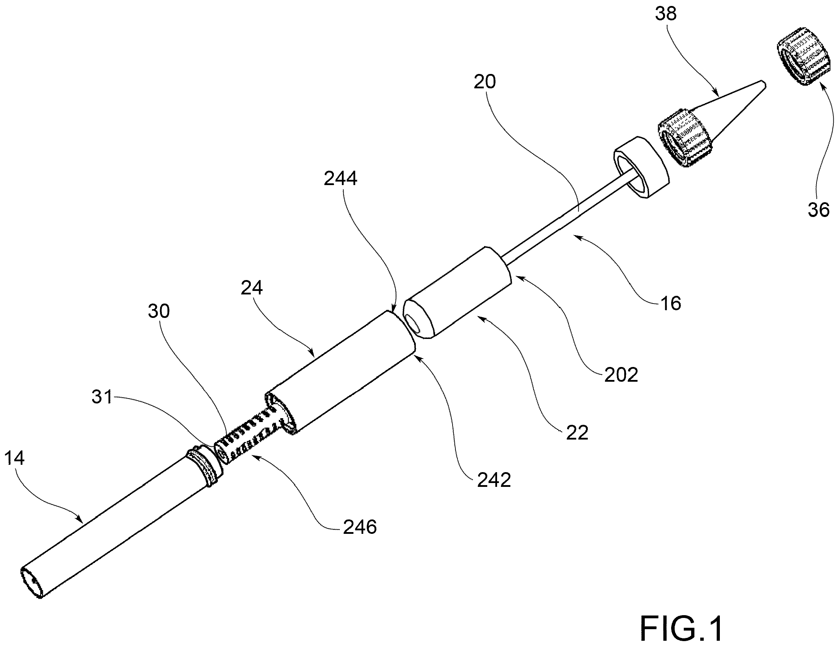

Fig. 1 shows a device according to the present invention which is indicated with the reference sign 12.

In its essential form, the device 12 for taking and handling a saliva sample comprises:
- a test tube 14 suitable for containing a sample of saliva;
- a collection element 16 comprising a stem 20 provided at a first end 202 with an absorbent element 22 suitable for absorbing a specified amount of saliva, and suitable for being squeezed to release at least part of the amount of saliva absorbed; and
- a collector 24 provided at a first end 242 with a mouth 244 of a shaped seat 26 suitable for allowing the insertion of the absorbent element 22, wherein the shaped seat 26 comprises a narrowing 28 suitable for allowing the squeezing of the absorbent element 22, and the collector at a second end 246 comprises at least one collection hole 30, 31.

The collector 24 is suitable for being at least partially inserted inside the test tube 14 so that the at least one collection hole 30, 31 is suitable for realizing a fluidic communication between the shaped seat 26 and the inside of the test tube 14.

According to a possible embodiment, at a second end 204 of the stem 20 of the collection element 16 a handling element 32 may be provided, projecting in a radial direction with respect to the stem 20. Preferably, the handling element 32 comprises a stop portion 34 for the mouth 244 of the collector 24.

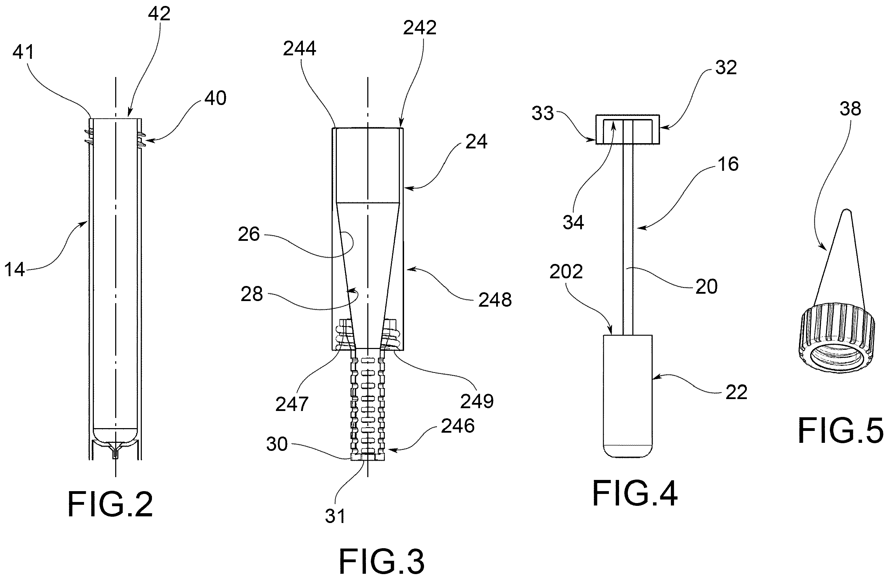

As may be seen in Fig. 4, the handling element 32 may comprise a substantially cylindrical outer element 33 which in use is intended to at least partially cover the collector 24.

Advantageously, the stop portion 34 may be an inner connecting surface 34 between the handling element and the substantially cylindrical outer element 33.

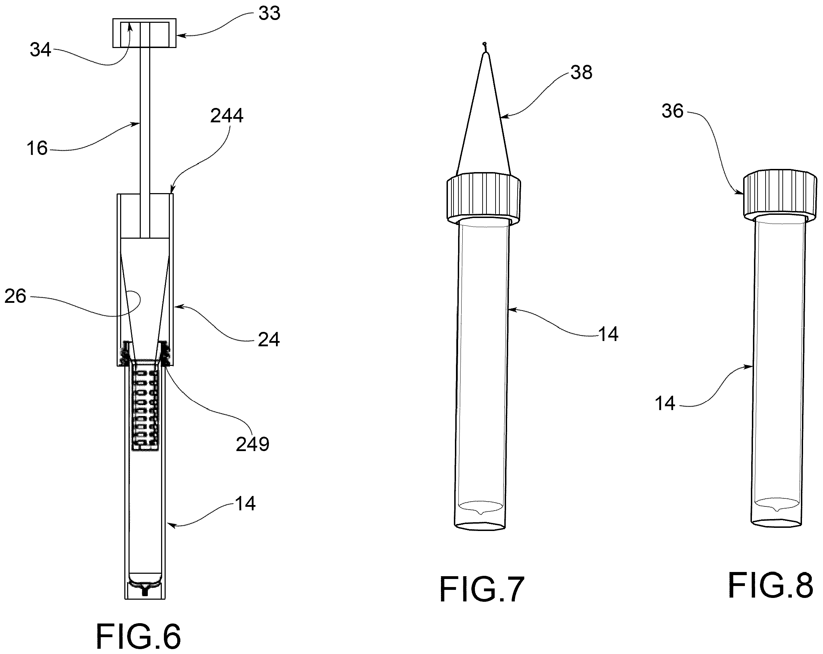

By virtue of the presence of the stop between the stop portion 34 and the mouth 244 of the collector 24, it is possible to make the results obtained from different saliva samples taken with a plurality of devices 12 repeatable and therefore comparable with each other. One such operating step is shown for example in the example of Fig. 6.

According to a possible embodiment, the absorbent element 22 may have a substantially cylindrical shape. As may be seen in the accompanying figures, the end of the absorbent element 22 may be rounded or tapered (solution not shown) to allow easier insertion of the absorbent element 22 inside the shaped seat 26 of the collector 24.

The absorbent element 22 may be made of porous sponge, preferably of polyurethane foam. In fact, as known, polyurethane foam is an inert material that allows the recovery of proteins, antibodies, peptides, metabolites, nucleic acids as well as microbial particles including viruses or bacteria or fungi.

The absorbent element 22 may be predisposed with sodium bicarbonate powder. Advantageously, the sodium bicarbonate powder may be suitable for facilitating the collection of the saliva sample.

According to a possible embodiment, the absorbent element 22 may have a volumetric absorption capacity of at least 1 ml. Advantageously, the absorbent element 22 may have a volumetric absorption capacity between 0.5 ml and 2.0 ml.

According to a possible embodiment, the collector 24 at the second end 246 may comprise a plurality of radial holes 30.

As may be seen in Fig. 3, the collector 24 at the second end 246 may comprise a plurality of radial holes 30 and at least one axial hole 31.

According to a possible embodiment, the portion of collector 24 provided with radial holes 30 may have a length between 25 mm and 45 mm, preferably around 35 mm.

According to a possible embodiment, the shaped seat 26 of the collector 24 for the collection element 16 may have a substantially conical shape with an initial diameter between 18 mm and 22 mm, and an end diameter between 6 mm and 10 mm. Preferably, the shaped seat 26 of the collector 24 for the collection element 16 may have a substantially conical shape with an initial diameter of about 20 mm, and an end diameter of about 8 mm.

In this embodiment, the narrowing 28 is achieved by providing a substantially cone-shaped seat.

The at least one radial hole 30 may have a substantially rectangular section, with the short side parallel to the axial direction.

As may be seen in Fig. 3, the collector 24 may comprise a radially projecting portion 247, which in use may be suitable for stopping against an insertion portion 42 of the test tube 14.

Advantageously, the collector 24 may be provided with an outer casing 248, suitable in use for at least partially covering the test tube 14. One such operating step is shown for example in Fig. 6.

According to a possible embodiment, a thread 249 suitable for coupling with a corresponding threaded portion 40 provided at an insertion portion 42 of the test tube 14 may be provided at an inner portion of the outer casing 248.

According to a possible embodiment, the device 12 may comprise a screw cap 36 suitable for screwing onto a corresponding threaded portion 40 provided at an insertion portion 42 of the test tube 14. Fig. 8 shows an embodiment in which the test tube 14 is closed with a stopper 36.

. The stopper 36 may also be provided with a central rubber portion, which allows the sampling of saliva by means of needle-punching systems. This ensures biological safety as it eliminates the need to open the test tube.

According to a possible embodiment, the device 12 may comprise a screw cap with dropper 38 suitable for screwing onto a corresponding threaded portion 40 provided at an insertion portion 42 of the test tube 14.

According to a possible embodiment, the test tube 14 may be at least partially filled with a viral inactivation buffer dried by evaporation or freeze-drying.

The method for taking and handling a saliva sample essentially comprises the steps of:
(a) providing a device 12 for taking and handling a saliva sample according to that described above;
(b) inserting the absorbent element 16 into the mouth of the user for a specified time;
(c) inserting the collection element 22 inside the shaped seat 26 of the collector 24 to a position that is such that the absorbent element 16 is squeezed, the collector 24 being at least partially inserted in said test tube 14;
(d) removing the collector 24 and the collection element 22 from the test tube 14.

According to a possible embodiment, in step (c) the insertion of the collection element 22 inside the shaped seat 26 of the collector 24 may continue until the stop portion 34 stops against the mouth 24.

Advantageously, the collector 24 may be pre-inserted inside the test tube 14.

According to a possible embodiment, the method may comprise a final step in which the test tube 14 is closed with a stopper 36, or with a screw cap with a dropper 38. According to a possible embodiment, the stem 20 of the handling element 32 of the collection element 16 may be made of polymeric material, of a per se known type.

Similarly, the collector may be made of polymeric material, of a per se known type.

The test tube may be made, for example, of polymeric material or glass, according to specific requirements.

The device for taking and handling a saliva sample was tested on a series of samples.

In a first evaluation, the molecular analysis for SARS-CoV-2 was carried out on a series of eight saliva samples collected both by means of a commercially available device and by means of the device of the present invention. Molecular analysis was performed by searching through the CE-IVD system for the sequences of three genes of the SARS-CoV-2 virus, the Orflab gene, the N gene and the S gene in a single analytical session. The analysis of the results shows that the saliva collection system object of the present invention allows a greater diagnostic sensitivity to be achieved for the search of the molecular sequences of SARS-CoV-2, as documented by the detection of a positive signal (Cycle threshold or Ct) lower than that of the same saliva sample collected with the device of the prior art. In a series of six samples, the SARS-CoV-2 antigen was also determined by chemiluminescence method. Also in this case, the collection device of the present invention has made it possible to increase the level of sensitivity for detecting the antigen. The analytical systems available on the market for this determination provide for the addition of viral inactivation buffers to the sample which causes dilution of the sample and consequent lower analytical sensitivity. Pre-filling the saliva collection test tube with a dried inactivation buffer will ensure safety and at the same time increased analytical sensitivity.

The advantages that may be achieved with the device and the method according to the present invention are thus now apparent.

First, the collector and the collection element may be removed and disposed of by the user. The components of the device that must be disposed of after collection (collector and collection element) may have a characteristic identifying color, for example yellow, and the components to be delivered to the laboratory (test tube and stopper) may have another color, for example white.

The test tube may then be closed with an airtight screw cap and may be safely transported to the analytical laboratory. Alternatively, the test tube may be closed with a screw cap with dropper which allows a subsequent controlled extraction of liquid and suitable for the use of a POC test.

The invention is applicable in all fields including the pediatric, disability and vulnerability fields.

The collection device and method are simple, easy and intuitive to perform, so as to allow autonomous use by all potential users including children.

Furthermore, the collection device and method are safe for the user as they do not involve any risk of accidental ingestion. It is therefore also suitable for children, the elderly and people with both physical and mental disabilities.

The collection system guarantees maximum safety with respect to biological risk in all the sample collection and processing steps.

Furthermore, the collection system is versatile as it may be used without further handling both for point-of-care (POC) analyzes and for analyzes on laboratory instruments, even with high automation.

The collection system allows any analytical typology to be applied to the sample including molecular, immunometric, mass spectrometry, immunochemical, immunoenzymatic and cellular analyzes.

A person skilled in the art may, in order to satisfy particular requirements, make modifications to the embodiments described above and/or substitute described elements with equivalent elements without thereby departing from the scope of the accompanying claims.

## Claims

1. Device (12) for taking and handling a saliva sample comprising:
- a test tube (14) suitable for containing a sample of saliva;
- a collection element (16) comprising a stem (20) provided at a first end (202) with an absorbent element (22) suitable for absorbing a specified amount of saliva, and suitable for being squeezed to release at least part of the amount of saliva absorbed; and
- a collector (24) provided at a first end (242) with a mouth (244) of a shaped seat (26) suitable to allow the insertion of said absorbent element (22), said shaped seat (26) comprising a narrowing (28) suitable for allowing the squeezing of said absorbent element (22); said collector (24) at a second end (246) comprising at least one collection hole (30, 31);
wherein said collector (24) is suitable to be at least partially inserted inside said test tube (14) such that said at least one collection hole (30, 31) is suitable to realize a fluidic communication between the shaped seat (26) and the inside of the test tube (14).

2. Device (12) according to claim 1, **characterized in that** at a second end (204) of said stem (20) of said collection element (16) a handling element (32) is provided projecting in a radial direction with respect to said stem (20), said handling element (32) comprising a stop portion (34) for the mouth (24) of the collector (24) .

3. Device (12) according to any of the preceding claims, **characterized in that** said absorbent element (22) has a substantially cylindrical shape and is made of porous sponge, preferably of polyurethane foam.

4. Device (12) according to any of the preceding claims, **characterized in that** said absorbent element (22) is predisposed with sodium bicarbonate powder.

5. Device (12) according to any of the preceding claims, **characterized in that** said absorbent element (22) has a volumetric absorption capacity of at least 1 ml.

6. Device (12) according to any of the preceding claims, **characterized in that** said collector (24) at the second end (246) comprises a plurality of radial holes (30) and at least one axial hole (31).

7. Device (12) according to any of the preceding claims, **characterized in that** the collector portion (24) provided with radial holes (30) has a length between 25 mm and 45 mm.

8. Device (12) according to any of the preceding claims, **characterized in that** the shaped seat (26) of said collector (24) for said collection element (16) has a substantially conical shape with an initial diameter that is between 18 mm and 22 mm, and an end diameter, representing said narrowing (28) with a diameter between 6 mm and 10 mm.

9. Device (12) according to any of the preceding claims, **characterized in that** said at least one radial hole (30) has a substantially rectangular cross-section, with short side parallel to the axial direction.

10. Device (12) according to any of the preceding claims, **characterized in that** said collector (24) is provided with an outer casing (248), suitable in use to cover the test tube (14) at least partially, at an inner portion of said outer casing (248) a thread (249) being provided suitable to couple with a corresponding threaded portion (40) provided at an insertion portion (42) of said test tube (14).

11. Device (12) according to any of the preceding claims, **characterized in that** it comprises a screw cap (36) suitable for screwing onto a corresponding threaded portion (40) provided at an insertion portion (42) of said test tube (14).

12. Device (12) according to any of the preceding claims, **characterized in that** it comprises a screw cap with dropper (38) suitable for screwing onto a corresponding threaded portion (40) provided at an insertion portion (42) of said test tube (14).

13. Device (12) according to any of the preceding claims, **characterized in that** said test tube (14) is at least partially filled with a viral inactivation buffer dried by evaporation or freeze-drying.

14. Method for taking and handling a saliva sample comprising the steps of:
(a) providing a device (12) for taking and handling a saliva sample according to any of the claims 1-13;
(b) inserting the absorbent element (16) into the mouth of the user for a specified time;
(c) inserting the collection element (22) inside the shaped seat (26) of said collector (24) to a position such that said absorbing element (16) is squeezed, said collector (24) being at least partially inserted in said test tube (14);
(d) removing said collector (24) and said collecting element (22) from said test tube (14).

15. Method according to the preceding claim, **characterized in that** in said step (c) the insertion of the collecting element (22) inside the shaped seat (26) of said collector (24) continues until the stop portion (34) stops against the mouth (24).

16. Method according to any of the claims 14-15, **characterized in that** said collector (24) is pre-inserted inside said test tube (14).

17. Method according to any of the claims from 14-16, **characterized in that** it comprises a final step in which said test tube (14) is closed with a stopper (36, 38).
